(19) Europäisches Patentamt / European Patent Office / Office européen des brevets

(11) **EP 3 502 293 B1**

(12) **EUROPEAN PATENT SPECIFICATION**

(45) Date of publication and mention of the grant of the patent:
**13.05.2020   Bulletin 2020/20**

(21) Application number: **17210463.0**

(22) Date of filing: **22.12.2017**

(51) Int Cl.:
*C22C 38/00* (2006.01)     *C22C 38/02* (2006.01)
*C22C 38/04* (2006.01)     *C22C 38/42* (2006.01)
*C22C 38/44* (2006.01)     *C22C 38/58* (2006.01)
*C22C 38/52* (2006.01)     *B01D 1/00* (2006.01)
*B01D 1/30* (2006.01)     *B01D 5/00* (2006.01)
*C21D 6/00* (2006.01)     *B01D 3/32* (2006.01)
*B01J 19/02* (2006.01)     *B22F 3/15* (2006.01)
*C07C 273/04* (2006.01)

(54) **USES OF DUPLEX STAINLESS STEELS**

VERWENDUNGEN VON DUPLEX EDELSTÄHLEN

UTILISATIONS DES ACIERS INOXYDABLES DUPLEX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.06.2019   Bulletin 2019/26**

(73) Proprietor: **Saipem S.p.A.**
**20097 San Donato Milanese (Milano) (IT)**

(72) Inventors:
 • **PACI, Giulio**
 **20097 SAN DONATO MILANESE (IT)**
 • **QUATTROCCHI, Mara**
 **20097 SAN DONATO MILANESE (IT)**
 • **CARLESSI, Lino**
 **20097 SAN DONATO MILANESE (IT)**
 • **SERRAFERO, Alberto**
 **20097 SAN DONATO MILANESE (IT)**

(74) Representative: **Cernuzzi, Daniele et al**
**Studio Torta S.p.A.**
**Via Viotti, 9**
**10121 Torino (IT)**

(56) References cited:
**WO-A1-2006/049572     WO-A1-2017/013180**
**US-A1- 2015 050 180**

**Description**

FIELD OF THE INVENTION

The present invention relates to the use of a duplex stainless steel in highly corrosive urea environments containing ammonia carbamate at high temperatures and pressures.

The invention thus also relates to the use of a duplex stainless steel in a urea plant (i.e. plant for production of urea), and specifically in an apparatus, equipment or device (or a part thereof) which is exposed to concentrated ammonium carbamate at high temperature.

The invention also relates to an apparatus, equipment or device of a urea production plant or used in a urea production process, comprising at least a part made of a corrosion resistant duplex stainless steel.

The invention also relates to a plant and a process for the production of urea comprising at least one apparatus, equipment or device having at least a part made of a duplex stainless steel, and to a method of revamping an existing urea production plant by replacing at least a part of an apparatus, equipment or device of the plant with a part made of a duplex stainless steel.

DESCRIPTION OF PRIOR ART

Duplex stainless steels are a family of stainless steels characterized by a two-phase microstructure consisting of grains of austenite and ferrite in roughly equal proportions.

The austenite-ferrite structure gives this family of stainless steels a combination of favorable properties, in particular good mechanical strength and excellent resistance to corrosion.

However, commonly available grades of duplex stainless steels, even if generally exhibiting a good corrosion resistance, are not suitable for use under very severe conditions, such as in a urea production plant and specifically in a high pressure section of a urea plant.

As it is known, urea production is based on a high-temperature, high-pressure reaction of carbon dioxide and ammonia to form ammonium carbamate, and a subsequent dehydration reaction of the ammonium carbamate to form urea and water.

In a typical urea production plant (urea plant), these processes are generally carried out in a urea synthesis reactor operating at high pressure and high temperature; the aqueous urea solution produced in the synthesis reactor is then progressively concentrated, with recover of unconverted reagents, in one or more recovery sections, for example in a high-pressure section, a medium-pressure section and a low-pressure section; finally, the urea is solidified in a finishing section, which normally includes a granulator or a prilling tower.

Industrial level processes and plants for the production of urea are today largely based on stripping processes: the synthesis solution exiting from the reactor is subjected to heating at high pressure (substantially the same pressure of the reactor) and the ammonium carbamate decomposes into ammonia and carbon dioxide in the liquid phase; part of the ammonia, together with carbon dioxide, passes from the liquid phase to the gas phase. The gas phase collected from the stripper is condensed and recycled to the reactor.

In some industrial processes, ammonia is used as a stripping agent (ammonia-stripping process), or the stripping is performed only by supplying heat, without any stripping agent (self-stripping process, or thermal stripping process).

In other industrial processes, such as the so-called CO2-stripping process, the stripping agent is gaseous carbon dioxide.

In a urea synthesis plant operating according to the ammonia-stripping process or the self-stripping process, corrosion resistance is an essential feature.

In particular, the ammonia-stripping process and the self-stripping process have a high pressure section, basically comprising the urea synthesis reactor and the urea stripper (as well as auxiliary equipment and devices), where the corrosion resistance is most important, due to the presence of the intermediate compound ammonium carbamate solution.

The ammonia-stripping process and the self-stripping process are in fact preferably performed at a maximum temperature of 185°C or higher (more preferably at 190°C or higher, in particular at 205°C or higher and preferably in the range 205-215°C); at a maximum pressure of 150 bar or higher (preferably of 156 bar or higher and more preferably of about 160 bar or higher); and with a NH3/CO2 molar ratio (so-called N/C ratio) in the range 3.2-3.6.

For example, stripping processes of the type described just above, operating at such conditions, are used in the so-called "Snamprogetti Urea Technology", which is well known to the skilled person being widely used worldwide and often cited in technical texts and papers.

Thus, at least some apparatus, equipment or device of the urea plant, in particular of the high pressure section thereof, such as (but not only) the urea stripper, operate under processing conditions which are highly corrosive, particularly due to the presence of a hot and concentrated carbamate solution at high temperatures (185°-205°C and over) and pressures (150 bar or higher).

Similar problems are however also present in other kinds of urea production plants also having a high pressure section.

Therefore, the high pressure section of a urea plant (in particular, but not only, in a urea plant operating according to the ammonia-stripping process or the self-stripping process) usually requires addition of a certain amount of oxygen (typically in form of a stream of inerts also including oxygen) for passivating the metal surfaces (especially, but not only, if made of austenitic stainless steels). Use of oxygen in the high pressure section can however increase the risk to originate potentially explosive mixture and therefore there is a concern in terms of safety.

In order to reduce the use of passivation gas streams and/or to improve corrosion resistance, duplex stainless steels have been proposed for use in urea production plants.

For example, WO95/00674 discloses the use of a particular duplex stainless steel, the so called super duplex stainless steel sold under the trademark Safurex®, for making some equipment of urea plants.

However, the super duplex stainless steels of WO95/00674, when used in a carbamate environment, may be not fully effective at very high temperatures (higher than 180-200°C), such as common operation temperatures of ammonia-stripping or self-stripping processes. Use of known duplex stainless steels is thus confined to CO2-stripping processes.

WO2014/180761 discloses a shell-and-tube urea stripper, to be specifically used in an ammonia-stripping or self-stripping process, having a bundle of tubes made of certain duplex stainless steels, namely of the Safurex® steel 29Cr-6.5Ni-2Mo-N (ASME Code 2295-3 and UNS S32906), or the DP28W™ steel 27Cr-7.6Ni-lMo-2.3W-N (ASME Code 2496-1 and UNS S32808) .

Also WO2017013180-A1, WO2017013181-A1 and WO2017014632-disclose duplex stainless steels generally suggested for use in urea plants under high temperature and high pressure conditions.

It can be appreciated that all the prior art documents cited above disclose duplex stainless steels which do not contain cobalt.

WO2006/049572 discloses a duplex stainless steel alloy which contains also cobalt and shows high strength, good corrosion resistance, good workability and which is weldable. The proposed alloys are intended for use in the onshore and offshore sectors of the oil and gas industry, while uses under more severe corrosive conditions (such as in a urea plant/process) are not mentioned.

US2015/050180A1 discloses a duplex ferritic austenitic stainless steel containing cobalt and which chemical composition and microstructure are favorable for use in chemical industries wherein good uniform corrosion resistance and high strength are required, such as for example in urea manufacturing.

Therefore, even if duplex stainless steels are known which have good corrosion resistance and are allegedly suitable for use also in a urea production plant, there is still a need for other, possibly more corrosion resistant duplex stainless steels which are suitable for use in any urea environments, i.e. in any kind of urea production plants/processes, and specifically in an apparatus operated at high temperatures in contact with very corrosive fluids (containing ammonium carbamate) and also under oxygen-free conditions, such as for instance (but not only) the high pressure strippers (operated at pressure of 150 bar and more) used in an ammonia-stripping process or a self-stripping process.

DISCLOSURE OF THE INVENTION

Accordingly, it is an object of the present invention to provide a duplex stainless steel suitable for overcoming the problem foregoing described of the prior art.

In particular, it is an object of the present invention to provide duplex stainless steels which are specifically and fully suitable to be used in a urea environment, i.e. in contact with a fluid comprising ammonium carbamate, such as a concentrated ammonium carbamate solution, and also at temperatures of at least 185°C, preferably of at least 190°C and more preferably of 205°C and more, even under oxygen-free conditions.

It is also a specific object of the invention to provide corrosion resistant duplex stainless steels which are suitable for use in any urea environments, i.e. in any kind of urea production plants/processes, and specifically in an apparatus (such as a high pressure stripper) used in an ammonia-stripping process or a self-stripping process, and thus operating at a maximum temperature of 185°C or higher (preferably at 190°C or higher, in particular at 205°C or higher and preferably in the range 205-215°C); and/or at a maximum pressure of 150 bar or higher (preferably of 156 bar or higher and more preferably of about 160 bar or higher); and/or with a NH3/CO2 molar ratio (so-called N/C ratio) in the range 3.2-3.6.

The present invention accordingly relates to a duplex stainless steel for use in a urea production plant and/or in a urea production process, as defined in Claim 1.

The invention also relates to an apparatus, equipment or device, in particular of a urea production plant or used in a urea production process, comprising at least a part made of a corrosion resistant duplex stainless steel, as defined in Claim 21.

The invention also relates to a plant and a process for the production of urea comprising at least one such apparatus, equipment or device having at least a part made of a duplex stainless steel as defined in Claims 22 and 23 respectively; and to a method of revamping an existing urea production plant by replacing at least a part of an apparatus, equipment or device of the plant with a part made of a duplex stainless steel as defined in Claim 24.

Advantageous preferred features of the invention are the subject matter of the dependent claims.

The duplex stainless steels of the invention are specifically characterized by a combination of Ni, Co and Mo: in fact, it has been recognized that such three elements, used together according to specific composition rules, have an unexpected combined effect on the corrosion resistance as well as on other favorable material properties.

In fact, it has been found that these three elements (Ni, Co, Mo) effectively increase corrosion resistance of a duplex stainless steel (having the particular composition of the invention) if each element is used in a specific content range and the contents of the three elements are linked to one another by a composition parameter Z which ranges between a minimum value $Z_{min}$ and a maximum value $Z_{max}$.

In particular, the duplex stainless steels of the invention have a composition parameter Z ranging between 14,95 and 19,80.

Composition parameter Z is a parameter representative of the combined contents of Ni, Co, Mo and defined by formula (I) :

$$Z = 1,062 \ (Ni+Co) + 4,185 \ Mo \qquad (I)$$

where Ni, Co, Mo indicate the weight percentage of Ni, Co, Mo respectively.

According to the invention:

$14,95 \leq Z \leq 19,80$

In other words, the inventors have found that duplex stainless steels having the particular compositions of the invention also exhibit an excellent corrosion resistance (in particular, in urea environments) if parameter Z is maintained in the ranges defined above, i.e. if components Ni, Co and Mo are used in amounts which satisfy formula (II):

$$Z_{min} \leq [1,062 \ (Ni+Co) + 4,185 \ Mo] \leq Z_{max} \qquad (II)$$

where:

Ni, Co, Mo indicate the weight percentage of Ni, Co, Mo respectively

$Z_{min} = 14,95$

$Z_{max} = 19,80$

Experimental test confirm that duplex stainless steels according to the invention, i.e. having a combined content of Ni, Co and Mo as previously defined, satisfying formula (II), have a corrosion rate in urea environments (containing ammonium carbamate) significantly lower than prior art materials, even at high temperature/pressure and in oxygen-free conditions.

Such a result cannot be expected in view of the prior art teachings.

It is in fact commonly recognized in the art (as reported by several scientific papers) that the content of nickel (Ni) in austenitic steels is detrimental under low oxygen condition.

Therefore, it is commonly understood that corrosion resistance of duplex stainless steels take advantage from a low content of nickel.

On the contrary, the inventors of the present invention have recognized that a certain amount of nickel, lower than in usual austenitic steels but higher than a minimum threshold, has indeed a good impact on corrosion resistance of a duplex stainless steel, if nickel is associated with cobalt (Co) and molybdenum (Mo) according to specific rules.

Specifically, the duplex stainless steels of the invention have a content of nickel ranging between 5,5% and 8%, preferably from 6,0% to 7,5% (here and below, all percentages are intended, if not otherwise specified, as weight percentages with respect to the total weight of the steel).

Nickel is in fact an austenite forming element and a certain amount of nickel is needed to maintain an equilibrium between ferrite and austenite phases. From the other hand, nickel has a negative impact on intermetallic precipitation.

According to the invention, cobalt is used in combination with nickel (and replacing part of the nickel) to obtain the required balance between ferrite and austenite phases and to improve corrosion resistance.

The inventors of the present invention have in fact realized that the content of nickel can be reduced by replacing nickel with cobalt, that works as a partial substitute and surprisingly also has the additional advantage of improving the corrosion resistance of the duplex stainless steels having the particular compositions of the invention.

Cobalt, in fact (unlike nickel), reduces the precipitations of intermetallic phases, strengthens the ferrite matrix and has a positive effect as austenite forming element.

Specifically, the duplex stainless steels of the invention have a content of Co in the range between 0.01% and 0,8%, preferably from 0,01% to 0,6%, more preferably from 0,02 to 0,6%, in particular from 0,04% to 0,6%.

According to the invention, the contents of nickel and cobalt is also linked to the content of molybdenum.

Molybdenum is a ferrite forming element which accelerates the precipitation of intermetallic phases especially in the presence of high levels of chromium (such as in the duplex stainless steels of the invention); therefore, the content of molybdenum should not exceed a maximum threshold.

On the other hand, a certain amount of molybdenum is beneficial for ammonium carbamate corrosion resistance and localised corrosion resistance, especially in the presence of ammonium carbamate and under oxygen-free conditions.

Specifically, molybdenum is in the range between 2% and 2,5%.

The features of the invention as previously defined also provides a method to design a duplex stainless steel for use in very corrosive environments, in particular in a urea plant/process.

In particular, the invention provides the rules for selecting an effective content of Ni, Co, Mo.

Once selected the content/amount of two out of the three components (Ni, Co, Mo), for example by taking into account the above technical considerations about expected effects of each individual elements, the content/amount of the third component is calculated by applying the relationships of the invention.

In addition to Ni, Co and Mo, the duplex stainless steels of the invention have a relatively high content of chromium (Cr), which increases corrosion resistance in ammonium carbamate solution environments, and at the same time allows a good microstructure without precipitation of third phases and a good hot workability.

Chromium has in fact a beneficial effect on corrosion resistance and allows higher process temperatures in urea production applications. Chromium is also beneficial for other types of corrosion such as pitting or crevice. On the other hand, high amounts of chromium increase the possibility of precipitation of intermetallic phases and are detrimental to hot workability. Therefore, the amount of chromium is higher than 30% but lower than 35%, preferably ranging between 30,5 and 35%, more preferably between 30,5 and 33%, even more preferably between 30,5 and 32%, in particular between 30,5 and 31,6%.

The duplex stainless steel of the invention may also contain the following elements:

Carbon (C). Carbon generally improves mechanical strength; however, according to the invention high contents of carbon are avoided in order to prevent precipitation of carbides. Therefore, the amount of carbon is not higher than 0,03%, preferably from 0,001% to 0,02%.

Silicon (Si). Silicon is used as a ferrite forming element and for deoxidization in the steel mill, i.e. in the manufacturing process of the duplex stainless steels. High amounts of silicon are avoided in order to reduce the possibility of precipitation of intermetallic phases. Thus the amount of silicon is not higher than 0,5%, preferably from 0,001% to 0,5%.

Manganese (Mn). Manganese increases the solubility of nitrogen (N), but has also a negative impact on corrosion resistance. Therefore, the amount of manganese is not higher than 2,5%, preferably from 0,5% to 2,2%, in particular from 1,0% to 2,2%.

Tungsten (W). Tungsten is a ferrite forming element. Tungsten also enhances general corrosion resistance. In particular, in the same way as Cr, Mo and N, also W increases pitting and crevice resistance. However, W accelerates the precipitation of intermetallic phases so its content is maintained below 2,5%, preferably from 0,001% to 2,5%, more preferably from 0,02% to 1%.

Nitrogen (N). Nitrogen is an austenite forming element. Nitrogen also enhances the microstructure stability delaying the precipitation of intermetallic phases and increases the strength of the metal matrix. Nitrogen is added also to increase the pitting and crevice corrosion resistance. For these reasons, at least 0,3% of nitrogen is used. On the other hand, higher contents of nitrogen would lead to poor hot workability, therefore the maximum value of N content is 0,6%. Thus, the content of N ranges from 0,3 to 0,6%, preferably from 0,35% to 0,6%, in particular from 0,4% to 0,6%.

Copper (Cu). Copper has in general a positive effect depressing the intermetallic precipitation kinetics, especially when relatively high amounts of Mo and W are present. However, for urea production applications copper is a harmful element because it forms complex ions with ammonia and deteriorates corrosion resistance. Therefore, Cu content is limited to a maximum of 1%, preferably from 0,001% to 0,9%, more preferably from 0,10 to 0,90% Cu and in particular from 0,10 to 0,40%.

Since the duplex stainless steels of the invention have a relatively high content of chromium (as well as nitrogen), hot workability could be negatively affected. In order to facilitate processing (in particular, hot forming) of the duplex stainless steels of the invention, one or more of the following elements are optionally added:

Calcium (Ca): 0,004% or less, preferably from 0,001% to 0,004%;

Magnesium (Mg): 0,004% or less, preferably from 0,001% to 0,004%;
One or more rare-earth elements: 0,1% or less, preferably 0,05% or less (total amount).

Preferably, the rare-earth elements are selected in the group consisting of Lanthanum (La), Cerium (Ce), Praseodymium (Pr) and mixtures thereof.

Rare-earth elements (metals) have very high deoxidation and desulphurization capacities and also decrease the average size of inclusions. They have a beneficial effect on hot workability based on the ability to combine with impurities that can segregate at grain boundaries (such as sulphur) and modify the shape and composition of the inclusions.

The steel compositions of the invention may also include unavoidable impurities such as Phosphorus (P) and Sulphur (S). The content of P and S should however be maintained as low as possible. In particular, high amounts of S are detrimental to hot workability. Thus, the S content should be less than 0,005% and the P content should be less than 0,025%. Typical amounts are less than 0,0005% for S and less than 0,020% for P.

The ferrite content of the duplex steel (austeno-ferritic alloy) according to the present invention is also of some importance for the corrosion resistance. According to some embodiments, therefore, the ferrite content ranges from 30% to 70% by volume, preferably from 35 to 60%vol., more preferably from 40 to 60%vol. The duplex stainless steels of the invention are suitably resistant to corrosion even when exposed to ammonium carbamate at high pressure (in particular, at a maximum pressure of 150 bar and higher, preferably of 156 bar and higher, more preferably of 160 bar and higher) and high temperature (in particular 185°C and higher, preferably 190°C and higher, more preferably 205°C and higher), and even in oxygen-free condition.

The invention thus provides improved formulations of duplex stainless steels, fully suitable for use in very corrosive conditions such as in a urea environment, i.e. in contact with a fluid comprising ammonium carbamate, also at temperatures of 185°C and more (and even at 205°C and more) and even under oxygen-free conditions.

In particular, the duplex stainless steels of the invention are intended for use in contact with ammonium carbamate solutions having a concentration of ammonium carbamate ranging from 15%w to 95%w, in particular from 50%w to 95%w; and/or at a temperature of 185°C or more, in particular of 190°C or more, in particular of 205°C or more).

The highly corrosion resistant duplex stainless steels of the invention are suitable for use in any urea environments, i.e. in any kind of urea production plants/processes, and specifically in apparatuses operated at high temperatures (185°C, 190°C but also 205°C and higher) in contact with fluids containing ammonium carbamate and also under oxygen-free conditions, such as for instance (but not only) the high pressure strippers used in the ammonia-stripping process or the self-stripping process.

Thus, the duplex stainless steels of the invention are especially useful for manufacturing equipment and devices (or parts thereof) which are exposed to concentrated ammonium carbamate at high temperature, such as parts of the heat exchanger tubes and/or, or for example, tubes of strippers.

The duplex stainless steels of the invention exhibit an excellent corrosion resistance in carbamate solutions (even in oxygen-free condition) also at temperature of 205°C and higher.

The materials of the invention are therefore suitable to be used in a urea production plant of any kinds, including in particular the most demanding conditions of an ammonia-stripping or self-stripping process.

The invention thus relates to the use of the duplex stainless steel as disclosed herein in a urea production plant, and specifically in an apparatus, equipment or device (or a part thereof) which is exposed to concentrated ammonium carbamate at high temperature.

The invention also relates to an apparatus, equipment or device, in particular of a urea production plant or used in a urea production process, comprising at least a part made of a corrosion resistant duplex stainless steel as disclosed herein.

The invention also relates to a plant and a process for the production of urea comprising at least one apparatus, equipment or device having at least a part made of a duplex stainless steel as disclosed herein; and to a method of revamping an existing urea production plant by replacing at least a part of an apparatus, equipment or device of the plant with a part made of a duplex stainless steel as disclosed herein.

As a result of the specific compositions of the duplex stainless steels of the invention, the following additional advantages are also achieved over the prior art, in particular in case of use in a high pressure apparatus of the urea plant:

- the corrosion rate in an piece of equipment (apparatus/device or part thereof) made of the duplex stainless steels of the invention drastically decreases with respect to a piece of equipment made of prior art materials;
- the need of passivation air is drastically reduced or even eliminated;
- the thickness of the apparatus/device, in particular of the high pressure piping loop, can be reduced, thus resulting in a significant reduction of the total weight and cost of the high pressure section, since the duplex stainless steels of the invention also have high mechanical characteristics;
- the temperature at the bottom of the stripper can be increased without increasing the corrosion rate;

- it is possible to avoid using, for the high pressure equipment, different materials with different features and prescription in terms of material specifications.

BRIEF DESCRIPTION OF THE DRAWINGS

In the appended drawings:

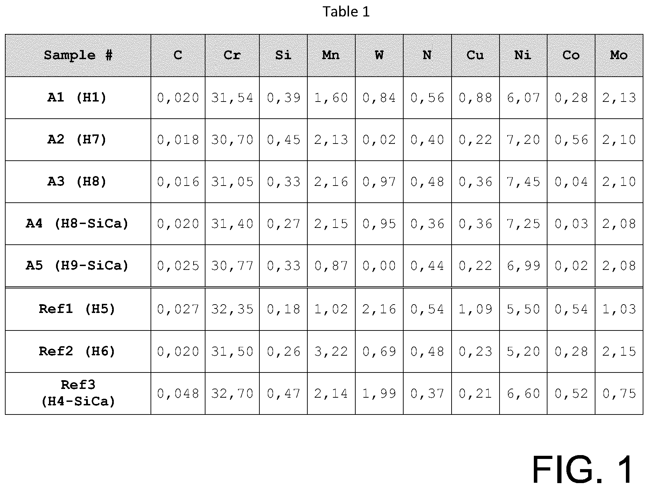

- figure 1 contains a table (Table 1) reporting the composition of exemplary samples of duplex stainless steels according to the invention, as well as of some reference samples;

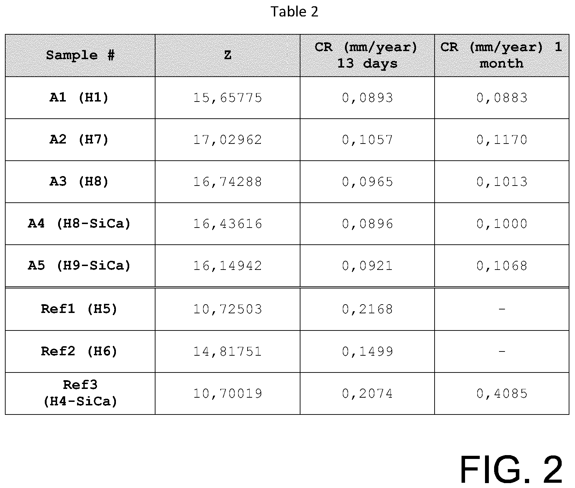

- figure 2 contains a table (Table 2) reporting the results of corrosion resistance tests performed on the samples of Table 1.

DETAILED DESCRIPTION OF PREFERRED EMBODIMENTS OF THE INVENTION

Duplex stainless steels according to the invention contains in weight % (%w):

C max 0,03
Si max 0,5
Mn max 2,5
Cr from more than 30,0 to 35,0
Ni 5, to 8,0
Co 0,01 to 0,8
Mo 2,0 to 2,5
W max 2,5
N 0,3-0,6
Cu max 1,0
Ca max 0,0040
Mg max 0,0040

one or more rare-earth elements max 0,1
the balance being Fe and impurities (as commonly understood, impurities are all those elements and compounds which are not purposively added to the steel formulation, but are however present in small amounts being contained in the raw materials used for manufacturing the duplex stainless steel).
The duplex stainless steels of the invention are further characterized in that the content of Ni, Co, Mo is such that:

$$Z_{min} \leq [1,062\ (Ni+Co) + 4,185\ Mo] \leq Z_{max} \qquad (II)$$

where:

Ni, Co, Mo indicate the weight percentage of Ni, Co, Mo respectively;
$Z_{min}$ = 14,95;
$Z_{max}$ = 19,80.

In other words, the duplex stainless steels of the invention have a composition parameter Z, representative of the combined contents of Ni, Co, Mo and defined by formula (I) :

$$Z = 1,062\ (Ni+Co) + 4,185\ Mo \qquad (I)$$

where Ni, Co, Mo indicate the weight percentage of Ni, Co, Mo respectively;
and wherein
$14,95 \leq Z \leq 19,80$.

Examples

Exemplary steel compositions according to the invention comprise, in percentages by weight:

C: 0,03% or less;
Si: 0,5% or less;
Mn: 2,5% or less;
Cr: 30,5% to 35%;
Ni: 5,5% to 8%;
Mo: 2% to 2,5%;
W: 0,02% to 1,0%;
Co: 0,01% to 0,8%;
N: 0,3% to 0,6%;
Cu: 1% or less;

one or more of the following:

Ca: 0,004% or less;
Mg: 0,004% or less;
one or more rare earth elements in a total amount of 0,05% or less;
the remainder being Fe and unavoidable impurities;
satisfying the relationship: $Z = 1{,}062 * (Ni + Co) + 4{,}185 * Mo$ is between 14,95 and 19,80.

Other embodiments of the steel of the invention comprise, in percentages by weight:

C: 0,001% to 0,03%;
Si: 0,001% to 0,5%;
Mn: 0,001% to 2,5%;
Cr: more than 30% to 35%;
Ni: 5,5% to 8%;
Mo: 2% to 2,5%;
W: 0,4% to 0,8%;
Co: 0,01% to 0,8%;
N: 0,3% to 0,6%;
Cu: 0,001% to 1%;

one or more of the following:

Ca: 0,001% to 0,004%;
Mg: 0,001% to 0,004%;
one or more rare earth elements in a total amount of 0,001% to 0,1%;
the remainder being Fe and unavoidable impurities;
satisfying the relationship: $Z = 1{,}062 * (Ni + Co) + 4{,}185 * Mo$ is between 14,95 and 19,80.

Other compositions according to the invention comprise, in percentages by weight:

C: 0,001% to 0,03%;
Si: 0,5% or less;
Mn: 0,5% to 2,2%;
Cr: 30,5% to 34%;
Ni: 5,5% to 8%;
Mo: 2% to 2,5%;
W: 2,5% or less;
Co: 0,01% to 0,8%;
N: 0,3% to 0,6%;
Cu: 1% or less;

one or more of the following:

Ca: 0,004% or less;
Mg: 0,004% or less;
one or more rare earth elements in a total amount of 0,05% or less;

the remainder being Fe and unavoidable impurities;
satisfying the relationship: Z = 1,062*(Ni + Co) + 4,185*Mo is between 14,95 and 19,80.

Yet other compositions according to the invention comprises, in percentages by weight:

C: 0,02% or less;
Si: 0,001% to 0,5%;
Mn: 2,5% or less;
Cr: 30,5% to 32%;
Ni: 5,5% to 8%;
Mo: 2% to 2,5%;
W: 0,1% to 1%;
Co: 0,01% to 0,8%;
N: 0,3% to 0,6%;
Cu: 0,15% to 0,25%;

having one or more of the following:

Ca: 0,004% or less;
Mg: 0,004% or less;
La, Ce, Pr or other rare earth elements: 0,05% or less
the remainder being Fe and unavoidable impurities;
satisfying the relationship CRC = 1,062*(Ni + Co) + 4,185*Mo is between 14,95 to 19,80.

Other compositions according to the invention comprises, in percentages by weight:

C: 0,03% or less;
Si: 0,5% or less;
Mn: 0,001% to 2,2%;
Cr: 31% to 35%;
Ni: 6% to 7,5%;
Mo: 2% to 2,5%;
W: 2,5% or less;
Co: 0,01% to 0,8%;
N: 0,4% to 0,6%;
Cu: 0,9% or less;

one or more of the following:

Ca: 0,004% or less;
Mg: 0,004% or less;
one or more rare earth elements in a total amount of 0,05% or less;
the remainder being Fe and unavoidable impurities;
satisfying the relationship: Z = 1,062*(Ni + Co) + 4,185*Mo is between 14,95 and 19,80.

Other examples according to this invention comprise, in percentages by weight:

C: 0,03% or less;
Si: 0,5% or less;
Mn: 0,5% to 2,2%;
Cr: 30,5% to 35%;
Ni: 5,5% to 6,5%;
Mo: 2% to 2,5%;
W: 0,001% to 2,5%;
Co: 0,01% to 0,6%;
N: 0,35% to 0,6%;
Cu: 1% or less;

one or more of the following:

Ca: 0,004% or less;
Mg: 0,004% or less;
one rare earth element selected from La, Ce, Pr or a combination thereof: 0,05% or less
the remainder being Fe and unavoidable impurities;
satisfying the relationship: $Z = 1,062*(Ni + Co) + 4,185*Mo$ is between 14,95 and 19,80.

For example, the present invention relates to elementary steel compositions that comprise, in percentages by weight:

C: 0,03% or less;
Si: 0,5% or less;
Mn: 2,2% or less;
Cr: 31% to 32%;
Ni: 5,5% to 8%;
Mo: 2% to 2,5%;
W: 2,5% or less;
Co: 0,02% to 0,4%;
N: 0,3% to 0,6%;
Cu: 0,001% to 1%;

one or more of the following:

Ca: 0,004% or less;
Mg: 0,004% or less;
one rare earth element selected from La, Ce, Pr or a combination thereof: 0,05% or less
the remainder being Fe and unavoidable impurities;
satisfying the relationship: $Z = 1,062*(Ni + Co) + 4,185*Mo$ is between 14,95 and 19,80.

Other exemplifying composition according to the invention comprise, in percentages by weight:

C: 0,03% or less;
Si: 0,5% or less;
Mn: 2% or less;
Cr: 30,5% to 33%;
Ni: 5,5% to 8%;
Mo: 2% to 2,5%;
W: 0,2% to 1%;
Co: 0,02% to 0,4%;
N: 0,3% to 0,6%;
Cu: 1% or less;

one or more of the following:

Ca: 0,001% to 0,004%;
Mg: 0,001% to 0,004%;
La, Ce, Pr or other rare earth elements: 0,001% to 0,05%
the remainder being Fe and unavoidable impurities;
satisfying the relationship: $Z = 1,062*(Ni + Co) + 4,185*Mo$ is between 14,95 and 19,80.

Further example compositions according to the invention comprise, in percentages by weight:

C: 0,02% or less;
Si: 0,5% or less;
Mn: 0,5% to 2,2%;
Cr: 30,5% to 34%;
Ni: 5,5 to 8%;

Mo: 2 to 2,5%;
W: 0,02 to 1%;
Co: 0,02 to 0,6%;
N: 0,3 to 0,6%;
Cu: 0,20% to 0,9%;

one or more of the following:

Ca: 0,004% or less;
Mg: 0,004% or less;
one or more rare earth elements in a total amount of 0,05% or less;
the remainder being Fe and unavoidable impurities;
satisfying the relationship: $Z = 1,062*(Ni + Co) + 4,185*Mo$ is between 14,95 and 19,80.

In particular, duplex stainless steels having the compositions in Table 1 were prepared and tested (in Table 1, some components are not indicated, being however in the amounts as previously disclosed).

The samples were prepared as common in the field and tested according to standard testing procedure.

In particular, corrosion tests were performed in a high pressure autoclave in ammonium carbamate solution at high pressure and high temperature (conditions representative of typical operation conditions in urea plants, in particular in the tubes of a urea stripper).

In particular, the corrosion resistance of the duplex stainless steels of the invention was tested in an oxygen-free carbamate solution, having a composition simulating the worst conditions normally occurring in the tubes of a high pressure section urea stripper of a urea plant, and at a temperature of 208°C.

In more detail: the corrosion behavior of the laboratory heats was checked via immersion tests that were conducted in a 5-liter Zirconium autoclave. The autoclave was equipped with adequate feed and discharge lines and a stirrer. The test solution contained a mixture of urea, ammonia and water, at concentrations similar to those of the urea synthesis process. Temperature and pressure for the experiments were set in the upper level of the typical ranges measured in a urea stripper, 180-210°C and 140-200 bar, respectively. The test solution was degassed before starting the tests to eliminate oxygen from the system. These experiments were designed to simulate the most severe conditions in a stripper of a urea plant without oxygen injection; note that under current working conditions in a urea plant, the stainless steel would perform even better, due to the presence of low amounts of oxygen and less aggressive conditions.

Test duration was 13 and 30 days. ASTM G31 (Standard Practice for Laboratory Immersion Corrosion Testing of Metals) standard indications were followed for test specimen preparation and the corrosion rate was measured by the gravimetric method.

After exposures of 13 days and 30 days respectively in the oxygen-free carbamate solution, the corrosion resistance was evaluated by calculating the corrosion rate (expressed in mm/year).

The results are shown in Table 2.

The results confirm that the samples (A1-A5) made of a duplex stainless steel according to the invention, i.e. satisfying the composition requirements of the invention (in particular with respect to the combined content of Ni, Co, Mo), have a corrosion rate significantly lower than comparative samples Ref1, Ref2, Ref3 and thus a better corrosion resistance.

In fact, the experimental tests confirm that when Z satisfies the requirement: $14.95 \leq Z \leq 19.80$, corrosion values are significantly lower than those exhibited by reference materials.

Corrosion values would be even significantly lower in working conditions in a urea plant, since the experimental set-up conditions are much more aggressive.

Finally, although the invention has been disclosed in relation to the above-mentioned preferred embodiments, it is to be understood that many other possible modifications and variations can be made without departing from the scope of the appended claims.

**Claims**

1.    Use of a duplex stainless steel in a urea production plant and/or in a urea production process, wherein the duplex stainless steel is used in a urea environment and in contact with a fluid comprising ammonium carbamate, and wherein the duplex stainless steel contains in weight percentage (%w):

C 0,03 or less
Si 0,5 or less

Mn 2,5 or less
Cr from more than 30,0 to 35,0
Ni from 5,5 to 8,0
Co from 0,01 to 0,8
Mo from 2,0 to 2,5
W 2,5 or less
N from 0,3 to 0,6
Cu 1,0 or less

having one or more of the following:

Ca 0,0040 or less
Mg 0,0040 or less
one or more rare-earth elements in a total amount of 0,1 or less;

the balance being Fe and impurities;
wherein the duplex stainless steel has a composition parameter (Z), representative of the combined contents of Ni, Co, Mo and defined by formula (I):

$$Z = 1,062 \ (Ni+Co) + 4,185 \ Mo \qquad (I)$$

where Ni, Co, Mo indicate the weight percentage of Ni, Co, Mo respectively;
said composition parameter (Z) ranging between 14,95 and 19,80.

2. The use according to claim 1, wherein the duplex stainless steel contains 30,5-35%w Cr.

3. The use according to one of the preceding claims, wherein the duplex stainless steel contains 30,5-32%w Cr, more preferably 30,5-31,6%w Cr.

4. The use according to one of the preceding claims, wherein the duplex stainless steel contains 0,10-0,90%w Cu or 0,10-0,40%w Cu.

5. The use according to one of the preceding claims, wherein the duplex stainless steel contains 0,02-0,6%w Co.

6. The use according to one of the preceding claims, wherein the duplex stainless steel contains 6,0-7,5%w Ni.

7. The use according to one of the preceding claims, wherein the duplex stainless steel contains Manganese from 0,5 to 2,2%w.

8. The use according to one of the preceding claims, wherein the duplex stainless steel contains Tungsten from 0,02 to 1,0%w.

9. The use according to one of the preceding claims, wherein the duplex stainless steel contains Calcium from 0,001 to 0,004%w.

10. The use according to one of the preceding claims, wherein the duplex stainless steel contains Magnesium (Mg) from 0,001 to 0,004%w.

11. The use according to one of the preceding claims, wherein the duplex stainless steel contains one or more rare-earth elements in a total amount of 0,05 or less.

12. The use according to one of the preceding claims, wherein the duplex stainless steel contains one or more rare-earth elements selected in the group consisting of Lanthanum (La), Cerium (Ce), Praseodymium (Pr) and mixtures thereof.

13. The use according to one of the preceding claims, wherein the duplex stainless steel contains, as impurities, no more than 0,025%w Phosphorus (P) and/or no more than 0,005%w Sulphur (S).

14. The use according to one of the preceding claims, wherein the duplex stainless steel is in contact with an ammonium carbamate solution having a concentration of ammonium carbamate ranging from 15%w to 95%w, in particular from 50%w to 95%w.

15. The use according to one of the preceding claims, wherein the duplex stainless steel is in contact with an ammonium carbamate solution at a temperature of 185°C or higher, preferably of 190°C or higher, more preferably of 205°C or higher.

16. The use according to one of the preceding claims, wherein the duplex stainless steel is used at a maximum temperature of 185°C or higher, preferably of 190°C or higher, more preferably of 205°C or higher, more preferably in the range 205-215°C; and/or at a maximum pressure of 150 bar or higher, preferably of 156 bar or higher and more preferably of 160 bar or higher; and/or in an environment having a NH3/CO2 molar ratio in the range 3,2-3,6.

17. The use according to one of the preceding claims, wherein the urea environment is under oxygen-free conditions.

18. The use according to one of the preceding claims, wherein the duplex stainless steel is used in a high pressure section of a urea plant.

19. The use according to one of the preceding claims, wherein the duplex stainless steel is used in an apparatus, equipment or device performing an ammonia-stripping process or a self-stripping process in a urea production process or plant.

20. The use according to one of the preceding claims, wherein the duplex stainless steel is used in a high pressure stripper configured for ammonia-stripping or self-stripping in a urea production process or plant.

21. An apparatus, equipment or device of a urea production plant or used in a urea production process, comprising at least a part which is exposed to concentrated ammonium carbamate at high temperature and is made of a corrosion resistant duplex stainless steel, wherein said duplex stainless steel contains in weight percentage (%w):

> C 0,03 or less
> Si 0,5 or less
> Mn 2,5 or less
> Cr from more than 30,0 to 35,0
> Ni from 5,5 to 8,0
> Co from 0,01 to 0,8
> Mo from 2,0 to 2,5
> W 2,5 or less
> N from 0,3 to 0,6
> Cu 1,0 or less

having one or more of the following:

> Ca 0,0040 or less
> Mg 0,0040 or less
> one or more rare-earth elements in a total amount of 0,1 or less;

the balance being Fe and impurities;
wherein the duplex stainless steel has a composition parameter (Z), representative of the combined contents of Ni, Co, Mo and defined by formula (I):

$$Z = 1,062 \ (Ni+Co) + 4,185 \ Mo \qquad (I)$$

where Ni, Co, Mo indicate the weight percentage of Ni, Co, Mo respectively;
said composition parameter (Z) ranging between 14,95 and 19,80.

22. A plant for the production of urea, comprising at least one apparatus, equipment or device according to claim 21.

23. A process for the production of urea comprising at least one step performed in an apparatus, equipment or device according to claim 21.

24. A method of revamping an existing urea production plant by replacing at least a part of an apparatus, equipment or device of the plant with a part made by the use according to one of the claims from 1 to 20.

**Patentansprüche**

1. Verwendung eines Duplex-Edelstahls in einer Harnstoff-Herstellungsanlage und/oder in einem Harnstoff-Herstellungsprozess, wobei der Duplex-Edelstahl in einer Harnstoffumgebung und in Kontakt mit einem Fluid verwendet wird, das Ammoniumcarbamat umfasst, und wobei der Duplex-Edelstahl in Gewichtsprozent (Gew.-%) enthält:

   C 0,03 oder weniger
   Si 0,5 oder weniger
   Mn 2,5 oder weniger
   Cr von mehr als 30,0 bis 35,0
   Ni von 5,5 bis 8,0
   Co von 0,01 bis 0,8
   Mo von 2,0 bis 2,5
   W 2,5 oder weniger
   N von 0,3 bis 0,6
   Cu 1,0 oder weniger

   und eines oder mehrere von Folgendem aufweist:

   Ca 0,0040 oder weniger
   Mg 0,0040 oder weniger;

   ein oder mehrere Seltenerdelemente in einer Gesamtmenge von 0,1 oder weniger;
   wobei der Rest Fe und Verunreinigungen sind;
   wobei der Duplex-Edelstahl einen Zusammensetzungsparameter (Z) aufweist, der für die kombinierten Gehalte an Ni, Co, Mo charakteristisch ist und durch die Formel (I) definiert ist:

   $$Z = 1,062\,(Ni+Co) + 4,185\,Mo \qquad (I)$$

   wo Ni, Co, Mo den Gewichtsprozentsatz von Ni, Co beziehungsweise Mo angeben;
   der Verbindungsparameter (Z) in einem Bereich zwischen 14,95 und 19,80 liegt.

2. Verwendung nach Anspruch 1, wobei der Duplex-Edelstahl 30,5 bis 35 Gew.-% Cr enthält.

3. Verwendung nach einem der vorhergehenden Ansprüche, wobei der Duplex-Edelstahl 30,5 bis 32 Gew.-% Cr, mehr zu bevorzugen 30,5 bis 31,6 Gew.-% Cr enthält.

4. Verwendung nach einem der vorhergehenden Ansprüche, wobei der Duplex-Edelstahl 0,10 bis 0,90 Gew.-% Cu oder 0,10 bis 0,40 Gew.-% Cu enthält.

5. Verwendung nach einem der vorhergehenden Ansprüche, wobei der Duplex-Edelstahl 0,02 bis 0,6 Gew.-% Co enthält.

6. Verwendung nach einem der vorhergehenden Ansprüche, wobei der Duplex-Edelstahl 6,0 bis 7,5 Gew.-% Ni enthält.

7. Verwendung nach einem der vorhergehenden Ansprüche, wobei der Duplex-Edelstahl von 0,5 bis 2,2 Gew.-% Mangan enthält.

8. Verwendung nach einem der vorhergehenden Ansprüche, wobei der Duplex-Edelstahl von 0,02 bis 1,0 Gew.-% Wolfram enthält.

9. Verwendung nach einem der vorhergehenden Ansprüche, wobei der Duplex-Edelstahl von 0,001 bis 0,004 Gew.-% Calcium enthält.

10. Verwendung nach einem der vorhergehenden Ansprüche, wobei der Duplex-Edelstahl von 0,001 bis 0,004 Gew.-% Magnesium (Mg) enthält.

11. Verwendung nach einem der vorhergehenden Ansprüche, wobei der Duplex-Edelstahl ein oder mehrere Seltenerdelemente in einer Gesamtmenge von 0,05 oder weniger enthält.

12. Verwendung nach einem der vorhergehenden Ansprüche, wobei der Duplex-Edelstahl ein oder mehrere Seltenerdelemente enthält, die in der Gruppe ausgewählt sind, die aus Lanthan (La), Cerium (Ce), Praseodym (Pr) und Mischungen davon besteht.

13. Verwendung nach einem der vorhergehenden Ansprüche, wobei der Duplex-Edelstahl als Verunreinigungen nicht mehr als 0,025 Gew.-% Phosphor (P) und/oder nicht mehr als 0,005 Gew.-% Schwefel (S) enthält.

14. Verwendung nach einem der vorhergehenden Ansprüche, wobei der Duplex-Edelstahl mit einer Ammoniumcarbamatlösung in Kontakt ist, die eine Konzentration an Ammoniumcarbamat in einem Bereich von 15 Gew.-% bis 95 Gew.-%, insbesondere von 50 Gew.-%, bis 95 Gew.-%, aufweist.

15. Verwendung nach einem der vorhergehenden Ansprüche, wobei der Duplex-Edelstahl mit einer Ammoniumcarbamatlösung bei einer Temperatur von 185 °C oder höher, vorzugsweise von 190 °C oder höher, mehr zu bevorzugen von 205 °C oder höher in Kontakt ist.

16. Verwendung nach einem der vorhergehenden Ansprüche, wobei der Duplex-Edelstahl bei einer Höchsttemperatur von 185 °C oder höher, vorzugsweise von 190 °C oder höher, noch mehr zu bevorzugen von 205 °C oder höher, mehr zu bevorzugen im Bereich von 205 bis 215 °C; und/oder bei einem Höchstdruck von 150 Bar oder höher, vorzugsweise von 156 Bar oder höher und mehr zu bevorzugen von 160 Bar oder höher; und/oder in einer Umgebung verwendet wird, die ein NH3/CO2-Molverhältnis im Bereich von 3,2 bis 3,6 aufweist.

17. Verwendung nach einem der vorhergehenden Ansprüche, wobei die Harnstoffumgebung sich unter sauerstofffreien Bedingungen befindet.

18. Verwendung nach einem der vorhergehenden Ansprüche, wobei der Duplex-Edelstahl in einem Hochdruckabschnitt einer Harnstoffanlage verwendet wird.

19. Verwendung nach einem der vorhergehenden Ansprüche, wobei der Duplex-Edelstahl in einer Einrichtung, Ausrüstung oder Vorrichtung verwendet wird, die einen Prozess zum Strippen von Ammoniak oder einen Prozess zum Selbst-Strippen in einem/einer Harnstoff-Herstellungsprozess oder -anlage ausführt.

20. Verwendung nach einem der vorhergehenden Ansprüche, wobei der Duplex-Edelstahl in einem Hochdruck-Stripper verwendet wird, der zum Strippen von Ammoniak oder zum Selbst-Strippen in einem/einer Harnstoff-Herstellungsprozess oder -anlage ausgestaltet ist.

21. Einrichtung, Ausrüstung oder Vorrichtung einer Harnstoff-Herstellungsanlage oder die in einem Hamstoff-Herstellungsprozess verwendet wird, die mindestens einen Teil umfasst, der konzentriertem Ammoniumcarbamat bei hoher Temperatur ausgesetzt wird und aus einem korrosionsbeständigen Duplex-Edelstahl hergestellt ist, wobei der Duplex-Edelstahl in Gewichtsprozent (Gew.-%) enthält:

C 0,03 oder weniger
Si 0,5 oder weniger
Mn 2,5 oder weniger
Cr von mehr als 30,0 bis 35,0
Ni von 5,5 bis 8,0
Co von 0,01 bis 0,8
Mo von 2,0 bis 2,5
W 2,5 oder weniger
N von 0,3 bis 0,6

Cu 1,0 oder weniger

und eines oder mehrere von Folgendem aufweist:

Ca 0,0040 oder weniger
Mg 0,0040 oder weniger

ein oder mehrere Seltenerdelemente in einer Gesamtmenge von 0,1 oder weniger;
wobei der Rest Fe und Verunreinigungen sind;
wobei der Duplex-Edelstahl einen Zusammensetzungsparameter (Z) aufweist, der für die kombinierten Gehalte an Ni, Co, Mo charakteristisch ist und durch die Formel (I) definiert ist:

$$Z = 1{,}062\,(Ni+Co) + 4{,}185\,Mo \qquad (I)$$

wo Ni, Co, Mo den Gewichtsprozentsatz von Ni, Co beziehungsweise Mo angeben,
der Verbindungsparameter (Z) in einem Bereich zwischen 14,95 und 19,80 liegt.

22. Anlage zur Herstellung von Harnstoff, die mindestens eine Einrichtung, Ausrüstung oder Vorrichtung nach Anspruch 21 umfasst.

23. Prozess zur Herstellung von Harnstoff, die mindestens eine Einrichtung, Ausrüstung oder Vorrichtung nach Anspruch 21 umfasst.

24. Verfahren zum Erneuern einer bestehenden Harnstoff-Herstellungsanlage durch Ersetzen von zumindest einem Teil einer Einrichtung, Ausrüstung oder Vorrichtung der Anlage mit einem Teil, das durch die Verwendung nach einem der Ansprüche 1 bis 20 hergestellt wird.

**Revendications**

1. Utilisation d'un acier inoxydable duplex dans une usine de production d'urée et/ou dans un processus de production d'urée, dans laquelle l'acier inoxydable duplex est utilisé dans un environnement d'urée et en contact avec un fluide comprenant du carbamate d'ammonium, et dans laquelle l'acier inoxydable duplex contient en pourcentage en poids (% en poids) :

0,03 ou moins de C
0,5 ou moins de Si
2,5 ou moins de Mn
de plus de 30,0 à 35,0 de Cr
de 5,5 à 8,0 de Ni
de 0,01 à 0,8 de Co
de 2,0 à 2,5 de Mo
2,5 ou moins de W
de 0,3 à 0,6 de N
1,0 ou moins de Cu

ayant un ou plusieurs des éléments suivants :

0,0040 ou moins de Ca
0,0040 ou moins de Mg

un ou plusieurs éléments des terres rares dans une quantité totale de 0,1 ou moins ;
le reste étant du Fe et des impuretés ;
dans laquelle l'acier inoxydable duplex a un paramètre de composition (Z), représentatif des teneurs combinées en Ni, Co, Mo et défini par la formule (I) :

$$Z = 1,062 \ (Ni + Co) + 4,185 \ Mo \qquad (I)$$

où Ni, Co, Mo indiquent respectivement le pourcentage en poids de Ni, Co, Mo ;
ledit paramètre de composition (Z) allant de 14,95 à 19,80.

2. Utilisation selon la revendication 1, dans laquelle l'acier inoxydable duplex contient 30,5 à 35 % en poids de Cr.

3. Utilisation selon l'une des revendications précédentes, dans laquelle l'acier inoxydable duplex contient 30,5 à 32 % en poids de Cr, de manière davantage préférée 30,5 à 31,6 % en poids de Cr.

4. Utilisation selon l'une des revendications précédentes, dans laquelle l'acier inoxydable duplex contient 0,10 à 0,90 % en poids de Cu ou 0,10 à 0,40 % en poids de Cu.

5. Utilisation selon l'une des revendications précédentes, dans laquelle l'acier inoxydable duplex contient 0,02 à 0,6 % en poids de Co.

6. Utilisation selon l'une des revendications précédentes, dans laquelle l'acier inoxydable duplex contient 6,0 à 7,5 % en poids de Ni.

7. Utilisation selon l'une des revendications précédentes, dans laquelle l'acier inoxydable duplex contient de 0,5 à 2,2 % en poids de manganèse.

8. Utilisation selon l'une des revendications précédentes, dans laquelle l'acier inoxydable duplex contient de 0,02 à 1,0 % en poids de tungstène.

9. Utilisation selon l'une des revendications précédentes, dans laquelle l'acier inoxydable duplex contient de 0,001 à 0,004 % en poids de calcium.

10. Utilisation selon l'une des revendications précédentes, dans laquelle l'acier inoxydable duplex contient de 0,001 à 0,004 % en poids de magnésium (Mg).

11. Utilisation selon l'une des revendications précédentes, dans laquelle l'acier inoxydable duplex contient un ou plusieurs éléments des terres rares dans une quantité totale de 0,05 ou moins.

12. Utilisation selon l'une des revendications précédentes, dans laquelle l'acier inoxydable duplex contient un ou plusieurs éléments des terres rares choisis dans le groupe consistant en le lanthane (La), le cérium (Ce), le praséodyme (Pr) et des mélanges de ceux-ci.

13. Utilisation selon l'une des revendications précédentes, dans laquelle l'acier inoxydable duplex contient, en tant qu'impuretés, pas plus de 0,025 % en poids de phosphore (P) et/ou pas plus de 0,005 % en poids de soufre (S).

14. Utilisation selon l'une des revendications précédentes, dans laquelle l'acier inoxydable duplex est en contact avec une solution de carbamate d'ammonium ayant une concentration en carbamate d'ammonium allant de 15 % en poids à 95 % en poids, notamment de 50 % en poids à 95 % en poids.

15. Utilisation selon l'une des revendications précédentes, dans laquelle l'acier inoxydable duplex est en contact avec une solution de carbamate d'ammonium à une température de 185 °C ou plus, de préférence de 190 °C ou plus, de manière davantage préférée de 205 °C ou plus.

16. Utilisation selon l'une des revendications précédentes, dans laquelle l'acier inoxydable duplex est utilisé à une température maximale de 185 °C ou plus, de préférence de 190 °C ou plus, de manière davantage préférée de 205 °C ou plus, de manière davantage préférée dans la plage de 205 à 215 °C ; et/ou à une pression maximale de 150 bar ou plus, de préférence de 156 bar ou plus et de manière davantage préférée de 160 bar ou plus ; et/ou dans un environnement ayant un rapport molaire $NH_3/CO_2$ dans la plage de 3,2 à 3,6.

17. Utilisation selon l'une des revendications précédentes, dans laquelle l'environnement d'urée est dans des conditions exemptes d'oxygène.

**18.** Utilisation selon l'une des revendications précédentes, dans laquelle l'acier inoxydable duplex est utilisé dans une section haute pression d'une usine d'urée.

**19.** Utilisation selon l'une des revendications précédentes, dans laquelle l'acier inoxydable duplex est utilisé dans un appareil, équipement ou dispositif réalisant un processus de stripage d'ammoniac ou un processus d'autostripage dans un processus ou une usine de production d'urée.

**20.** Utilisation selon l'une des revendications précédentes, dans laquelle l'acier inoxydable duplex est utilisé dans un dispositif de stripage haute pression configuré pour le stripage d'ammoniac ou l'autostripage dans un processus ou une usine de production d'urée.

**21.** Appareil, équipement ou dispositif d'une usine de production d'urée ou utilisé dans un processus de production d'urée, comprenant au moins une partie qui est exposée à du carbamate d'ammonium concentré à une température élevée et est réalisée en un acier inoxydable duplex résistant à la corrosion, dans lequel ledit acier inoxydable duplex contient en pourcentage en poids (% en poids) :

> 0,03 ou moins de C
> 0,5 ou moins de Si
> 2,5 ou moins de Mn
> de plus de 30,0 à 35,0 de Cr
> de 5,5 à 8,0 de Ni
> de 0,01 à 0,8 de Co
> de 2,0 à 2,5 de Mo
> 2,5 ou moins de W
> de 0,3 à 0,6 de N
> 1,0 ou moins de Cu

ayant un ou plusieurs des éléments suivants :

> 0,0040 ou moins de Ca
> 0,0040 ou moins de Mg

un ou plusieurs éléments des terres rares dans une quantité totale de 0,1 ou moins ;
le reste étant du Fe et des impuretés ;
dans lequel l'acier inoxydable duplex a un paramètre de composition (Z), représentatif des teneurs combinées en Ni, Co, Mo et défini par la formule (I) :

$$Z = 1,062 \ (Ni + Co) + 4,185 \ Mo \qquad (I)$$

où Ni, Co, Mo indiquent respectivement le pourcentage en poids de Ni, Co, Mo ;
ledit paramètre de composition (Z) allant de 14,95 à 19,80.

**22.** Usine pour la production d'urée, comprenant au moins un appareil, équipement ou dispositif selon la revendication 21.

**23.** Processus pour la production d'urée comprenant au moins une étape réalisée dans un appareil, équipement ou dispositif selon la revendication 21.

**24.** Procédé de modernisation d'une usine de production d'urée existante en remplaçant au moins une partie d'un appareil, équipement ou dispositif de l'usine par une partie réalisée par l'utilisation selon l'une des revendications 1 à 20.

Table 1

| Sample # | C | Cr | Si | Mn | W | N | Cu | Ni | Co | Mo |
|---|---|---|---|---|---|---|---|---|---|---|
| A1 (H1) | 0,020 | 31,54 | 0,39 | 1,60 | 0,84 | 0,56 | 0,88 | 6,07 | 0,28 | 2,13 |
| A2 (H7) | 0,018 | 30,70 | 0,45 | 2,13 | 0,02 | 0,40 | 0,22 | 7,20 | 0,56 | 2,10 |
| A3 (H8) | 0,016 | 31,05 | 0,33 | 2,16 | 0,97 | 0,48 | 0,36 | 7,45 | 0,04 | 2,10 |
| A4 (H8-SiCa) | 0,020 | 31,40 | 0,27 | 2,15 | 0,95 | 0,36 | 0,36 | 7,25 | 0,03 | 2,08 |
| A5 (H9-SiCa) | 0,025 | 30,77 | 0,33 | 0,87 | 0,00 | 0,44 | 0,22 | 6,99 | 0,02 | 2,08 |
| Ref1 (H5) | 0,027 | 32,35 | 0,18 | 1,02 | 2,16 | 0,54 | 1,09 | 5,50 | 0,54 | 1,03 |
| Ref2 (H6) | 0,020 | 31,50 | 0,26 | 3,22 | 0,69 | 0,48 | 0,23 | 5,20 | 0,28 | 2,15 |
| Ref3 (H4-SiCa) | 0,048 | 32,70 | 0,47 | 2,14 | 1,99 | 0,37 | 0,21 | 6,60 | 0,52 | 0,75 |

FIG. 1

Table 2

| Sample # | z | CR (mm/year) 13 days | CR (mm/year) 1 month |
|---|---|---|---|
| A1 (H1) | 15,65775 | 0,0893 | 0,0883 |
| A2 (H7) | 17,02962 | 0,1057 | 0,1170 |
| A3 (H8) | 16,74288 | 0,0965 | 0,1013 |
| A4 (H8-SiCa) | 16,43616 | 0,0896 | 0,1000 |
| A5 (H9-SiCa) | 16,14942 | 0,0921 | 0,1068 |
| Ref1 (H5) | 10,72503 | 0,2168 | - |
| Ref2 (H6) | 14,81751 | 0,1499 | - |
| Ref3 (H4-SiCa) | 10,70019 | 0,2074 | 0,4085 |

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9500674 A
- WO 2014180761 A
- WO 2017013180 A1
- WO 2017013181 A1
- WO 2017014632 A
- WO 2006049572 A
- US 2015050180 A1